# EUROPEAN PATENT APPLICATION

(11) **EP 2 159 219 A2**
(43) Date of publication of application: **03.03.2010**
(21) Application number: 09011543.7
(22) Date of filing: 01.03.2007
(51) Int. Cl.: C07C 315/06, C07C 317/44

(54) **An improved process for the preparation of armodafinil**

(30) Priority: 01.03.2006 US 778334 P; 23.03.2006 US 785812; 28.12.2006 US 878097 P
(62) Divisional of application: 07752169.8
(71) Applicant: Teva Pharmaceutical Industries Ltd., 49131 Petah Tiqva (IL)
(72) Inventor: Moshkovits-Kaptsan, Rinat, Raanana (IL); Braude, Viviana, 60920 Kadima (IL); Chen, Kobi, Kfar-saba (IL); Adler, Miri, Petach Tikva (IL)
(74) Representative: King, Lawrence

(57) **Abstract**

The invention encompasses a process for reducing the amount of bis(diphenylmethyl)disulfide in armodafinil, which comprises suspending armodafinil in a C₅ to C₁₂ hydrocarbon.

## Description

### RELATED APPLICATIONS

The application claims the benefit of U.S. provisional application Nos. 60/778,334, filed March 1, 2006; 60/785,812, filed March 23, 2006; and 60/878,097, filed December 28, 2006, hereby incorporated by reference.

### FIELD OF THE INVENTION

The invention encompasses processes for preparing intermediates of armodafinil, and the conversion of the intermediates to armodafinil.

### BACKGROUND OF THE INVENTION

Modafinil is currently marketed by Cephalon, Inc. under the trade name PROVIGIL^{®} as a racemic mixture of its R and S enantiomers. PROVIGIL^{®} is indicated for the treatment of excessive sleepiness associated with narcolepsy, shift work sleep disorder ("SWSD"), and obstructive sleep apnea/hypopnea syndrome ("OSA/HS").

Studies have shown that while both enantiomers of modafinil are pharmacologically active, the S enantiomer is eliminated from the body three times faster than the R enantiomer. Prisinzano et al., Tetrahedron: Asymmetry, vol. 5 (2004) 1053-1058. It is, therefore, preferable to develop pharmaceutical compositions of the R enantiomer of modafinil, as opposed to its racemic mixture.

The R enantiomer of modafinil is known as armodafinil and has the chemical name 2-[(R)-(diphenylmethyl)sulfinyl]acetamide. The molecular weight of armodafinil is 273.34 and it has the following chemical structure: 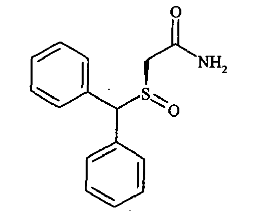 Armodafinil is commercially available as NUVIGIL^{™}. Armodafinil was well tolerated and the safety profile was comparable with modafinil.

Armodafinil was first disclosed in the U.S. Patent No. 4,927,855 ("'855 patent") and its EP parallel patent No. 0233106, both which were originally assigned to Lafon Laboratories.

The preparation of armodafinil is also disclosed in the '855 patent, and it is summarized in the following scheme: 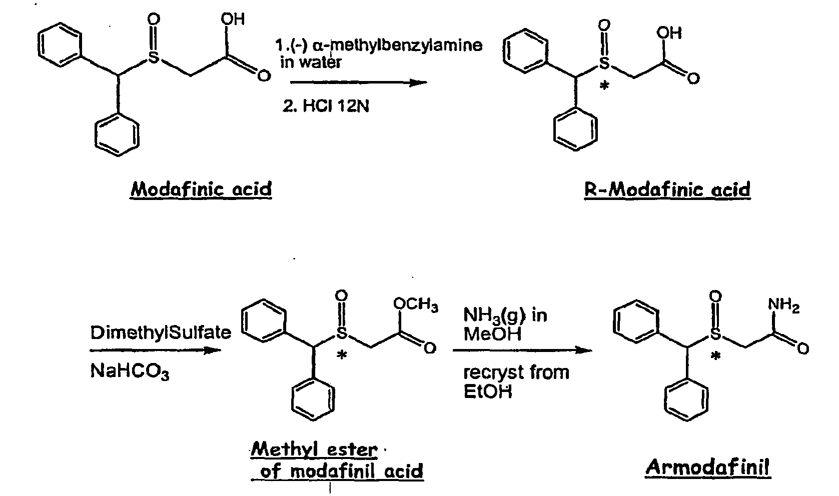 *See* '855 patent, col. 2,11. 16-53.

The process disclosed in the '855 patent uses (-)α-methylbenzylamine in the resolution step. Furthermore, two additional crystallization steps are performed in order to increase the enantiomeric purity of the modafinic acid. As a consequence, the overall yield reported in the '855 patent is rather unsatisfactory.

Other methods for performing an enantiomeric resolution are known in the art. For example, isolation of armodafinil from racemic modafinil can be performed by chromatography (such as High Performance Liquid Chromatography ("HPLC") or Simulated Moving Bed). Several publications also disclose resolution at the modafinic acid step by techniques such as preferential crystallization. *See, e.g.,* PCT publication No. WO 04/060858.

Osorio-Lozada et al., in Tetrahedron: Asymmetry (2004), 15(23), 3811-3815, describe the preparation of armodafinil by reacting racemic β-sulfinyl carboxylic acid with (R)-phenylthiazolidinethione, separating the diastereomers by column chromatography, and preparing armodafinil from the appropriate diastereomer. Although the diastereomers of this process are easier to separate by column chromatography than the enantiomers of modafinil, the amine required is not suitable for industrial-scale use and subsequent hydrolysis is necessary to yield armodafinil.

U.S. publication No. 2005/0154603 discloses the difficulty in transforming modafinic acid into modafinil: "[t]he convention method of chemical activation of the carboxylic group, *i.e.,* the transformation of the acid into the corresponding chloride is not compatible with the sulfoxide groups (see Oae "Organic Chemistry of Sulfur" Plenum Press N.Y. 1977 page 406), which would be reduced to sulfide under these conditions. See U.S. publication No. 2005/0154603, p. 3, ¶ [0022].

Asymmetric synthesis without the need for enantiomeric separation is disclosed in U.S. publication No. 2005/0080256 ('256 publication). The '256 publication describes the preparation of a sulfoxide as a single enantiomer or in an enantiomerically-enriched form by contacting a pro-chiral sulfide with a metal chiral complex, a base, and an oxidizing agent in an organic solvent. *See* '256 publication, pp. 3-4, ¶¶ 40-56. For example, Ph₂CHSCH₂CONH₂ was oxidized with cumene hydroperoxide in the presence of (S, S)-(-)-diethyl tartrate, Ti(OCHMe₂)₄, water, and diisopropyl ethyl amine in toluene to give 88.4% (-)-modafinil in < 99.5 % enantiomeric excess. *See id.* at p. 14, Example 1. The improvement of the enantiomeric purity of (-)modafinil is achieved by recrystallization. Because of the complexity of this procedure, however, it would be difficult to implement on an industrial scale.

Resolution of modafinic acid remains the simplest way to prepare armodafinil without the need to use sophisticated equipment, such as in preparative HPLC or SMB (Simulated Moving Bed), or expensive and sensitive reagents, such as in asymmetric oxidation. And even though some methods are available for enantiomeric resolution of modafinic acid, there remains a need in the art for more efficient methods with simple conditions that provide high yields.

Also, there is a need in the art for an easier and more efficient process for the conversion of modafinic acid to the ester intermediate in the preparation of armodafinil. Current processes use reagents that may not be suitable for high scale preparation. For example, the esterification step described in the '855 patent uses dimethyl sulfate, which is a toxic material. Alternative methods for the esterification step use iodomethane (Osorio-Lozada *et al.*) or haloformate (WO 04/060858), both of which are inappropriate for use on an industrial scale. Iodomethane is a toxic compound, suspected to be carcinogenic, and haloformates are corrosive and irritants. U.S. publication No. 2005/0282821 describes an esterification process for modafinil analogues, which is performed in H₂SO₄. However, use of sulfuric acid results in unsatisfactory conversion and unsatisfactory purity.

Like any synthetic compound, armodafinil can contain extraneous compounds or impurities that can come from many sources. They can be unreacted starting materials, by-products of the reaction, products of side reactions, or degradation products. Impurities in armodafinil, as an active pharmaceutical ingredient ("API"), are undesirable and, in extreme cases, might even be harmful to a patient being treated with a dosage form containing the API.

It is also known in the art that impurities in an API may arise from degradation of the API itself, which is related to the stability of the pure API during storage, and the manufacturing process, including the chemical synthesis. Process impurities include, but are not limited to, unreacted starting materials, chemical derivatives of impurities contained in starting materials, synthetic by-products, and degradation products.

In addition to stability, which is a factor in the shelf life of the API, the purity of the API produced in the commercial manufacturing process is clearly a necessary condition for commercialization. Impurities introduced during commercial manufacturing processes must be limited to very small amounts, and are preferably substantially absent. For example, the ICH Q7A guidance for API manufacturers requires that process impurities be maintained below set limits by specifying the quality of raw materials, controlling process parameters, such as temperature, pressure, time, and stoichiometric ratios, and including purification steps, such as crystallization, distillation, and liquid-liquid extraction, in the manufacturing process. *See* ICH Good Manufacturing Practice Guide for Active Pharmaceutical Ingredients, Q7A, Current Step 4 Version (November 10, 2000).

The product mixture of a chemical reaction is rarely a single compound with sufficient purity to comply with pharmaceutical standards. Side products and by-products of the reaction and adjunct reagents used in the reaction will, in most cases, also be present in the product mixture. At certain stages during processing of an API, such as armodafinil, it must be analyzed for purity, typically by high performance liquid chromatography ("HPLC"), nuclear magnetic resonance spectroscopy ("NMR"), or thin-layer chromatography ("TLC") analysis, to determine if it is suitable for continued processing and, ultimately, for use in a pharmaceutical product. The API need not be absolutely pure, as absolute purity is a theoretical ideal that is typically unattainable. Rather, purity standards are set with the intention of ensuring that an API is as free of impurities as possible, and, thus, as safe as possible for clinical use. In the United States, the Food and Drug Administration ICH Q7A guidelines recommend that the amounts of some impurities be limited to less than 0.1 percent. *See id.*

Generally, side products, by-products, and adjunct reagents (collectively "impurities") are identified spectroscopically and/or with another physical method, and then associated with a peak position, such as that in a chromatogram, or a spot on a TLC plate. *See* Strobel, H.A., et al., CHEMICAL INSTRUMENTATION: A SYSTEMATIC APPROACH, 953, 3d ed. (Wiley & Sons, New York 1989). Thereafter, the impurity can be identified, e.g., by its relative position in the chromatogram, where the position in a chromatogram is conventionally measured in minutes between injection of the sample on the column and elution of the particular component through the detector. The relative position in the chromatogram is known as the "retention time."

Thus, because of its medical uses, it is desirable to obtain armodafinil substantially free of certain impurities.

There is a need in the art for armodafinil having low levels of modafinic acid, and for processes of preparing armodafinil having a lower level of modafinic acid.

### SUMMARY OF THE INVENTION

One embodiment of the invention encompasses a process for the optical resolution of racemic modafinic acid comprising crystallization of racemic modafinic acid with (R)-α-naphthylethylamine.

Another embodiment of the invention encompasses R-modafinic acid containing less than about 2% area by HPLC of S-modafinic acid.

Yet another embodiment of the invention encompasses a process for preparing an (R)-C₁₋₂ alkyl ester ofmodafinic acid comprising combining R-modafinic acid, at least one acidic reagent, and a solvent selected from the group consisting of: methanol/aromatic hydrocarbon, methanol/ester, methanol and ethanol.

One embodiment of the invention encompasses an one-pot process for preparing armodafinil comprising esterification of R-modafinic acid according to the above process to form an (R)-C₁₋₂ alkyl ester of modafinic acid and reacting the (R)-C₁₋₂ alkyl ester of modafinic acid with ammonia or ammonium hydroxide to obtain armodafinil.

Another embodiment of the invention encompasses armodafinil containing less than about 0.5% area by HPLC of S-modafinil enantiomer.

Yet another embodiment of the invention encompasses armodafinil containing less than about 0.1% by HPLC area of modafinic acid.

One embodiment of the invention encompasses a process for reducing the amount of modafinic acid in armodafinil comprising contacting a solution of armodafinil with activated basic or activated neutral alumina.

Another embodiment of the invention encompasses armodafinil containing less than about 0.2% area by HPLC of R-MDF-DS.

Yet another embodiment of the invention encompasses a process for preparing R-MDF-DS comprising combining diphenylmethanthiol acetate, sodium methoxide and N,N-dimethylformamide to obtain a suspension, maintaining, and adding water.

One embodiment of the invention encompasses a process for reducing the amount of R-MDF-DS in armodafinil comprising suspending it in C₅ to C₁₂ hydrocarbon.

Another embodiment of the invention encompasses a process for preparing R-MDF-DS comprising combining diphenylmethanthiol acetate, sodium methoxide and N,N-dimethylformamide to obtain a suspension, maintaining, and adding water.

Yet another embodiment of the invention encompasses a pharmaceutical composition comprising a therapeutically effective amount of armodafinil and a pharmaceutically acceptable excipient, wherein the armodafinil contains less than about 0.1% area by HPLC of modafinic acid.

Another embodiment of the invention encompasses pharmaceutical compositions comprising a therapeutically effective amount of armodafinil and a pharmaceutically acceptable excipient, wherein the armodafinil contains less than about 0.5% area by HPLC of S-modafinil.

Yet another embodiment of the invention encompasses pharmaceutical compositions comprising a therapeutically effective amount of armodafinil and a pharmaceutically acceptable excipient, wherein the armodafinil contains less than about 0.2% area by HPLC of R-MDF-DS.

### DETAILED DESCRIPTION OF THE INVENTION

The prior art processes for preparing enantiomerically pure armodafinil via a modafinic acid ester intermediate generally produce low yields of product and employ equipment and reagents that are not suitable for use on an industrial scale. The invention provides an improved process for preparing enantiomerically pure armodafinil via a modafinic acid ester intermediate that produces high yields of product and is suitable for use on an industrial scale. As used herein, unless otherwise defined the term "BHSO" refers to modafinic acid.

This process provides R-modafinic acid in higher purity than the processes of the prior art. Example 1 repeated the process with (-)α-methylbenzylamine used in the '855 patent and various solvents, which yielded R-modafinic acid having relatively high contents of the undesired S-enantiomer of modafinic acid. For example, the repetition of the method of the '855 patent yielded the undesired S-enantiomer in 18.7%, 35.78%, and 18.44% area by HPLC, as shown in Examples 1 and 2 below.

Performing this process under different conditions, such as with different amines, also proved ineffective, as high levels of the S-modafinic acid were present in the desired R-enantiomer. For instance, using L-Proline as in Example 3 yielded the undesired S-enantiomer in 19.7% area by HPLC. Even when different solvents were used, the R-enantiomer remained enantiomerically impure. Solvents such as water and acetone yielded 49.9% and 18.6% area by HPLC, respectively, as shown in Example 4 below. Additionally, when the S-enantiomer of the amine was used instead of (R)-α-naphthylethylamine, the obtained product was the undesired S-modafinic acid, as shown in Example 5 below.

The invention encompasses processes for preparing armodafinil and intermediates thereof that are enantiomerically pure. As used herein, the term "enantiomerically pure" refers to an enantiomerically enriched composition of modafinic acid, modafinil, or armodafinil where a first enantiomer either R or S is present in an amount larger than a second enantiomer S or R. Preferably, enantiomerically pure refers to a composition having about 98% of one enantiomer, more preferably, the term refers to a composition having about 99% of one enantiomer, and most preferably, the term refers to a composition having about 99.9% of one enantiomer. The amount of enantiomer is determined by the HPLC methods described in the example section below.

One embodiment the invention encompasses a process for the optical resolution of racemic modafinic acid comprising crystallization of racemic modafinic acid with (R)-α-naphthylethylamine. The process comprises: (a) crystallization of racemic modafinic acid with (R)-α-naphthylethylamine to obtain (R)-modafinic acid*(R)-α-naphthylethylamine salt; and (b) converting the (R)-modafinic acid*(R)-α-naphthylethylamine salt to (R)-modafinic acid. Preferably, the crystallization is done in the presence of acetone. Preferably, step (a) comprises combining racemic modafinic acid, (R)-α-naphthylethylamine and acetone to form a solution, and cooling the solution to obtain (R)-modafinic acid*(R)-α-naphthylethylamine salt as a precipitate, and optionally isolating the (R)-modafinic acid*(R)-α-naphthylethylamine salt. Preferably, the process comprises combining racemic modafinic acid with (R)-α-naphthylethylamine and acetone to obtain a solution, cooling the solution to obtain a precipitate of (R)-modafinic acid*(R)-α-naphthylethylamine salt; and adding an acid. Optionally, the cooling step can include stirring. Preferably, the process is performed at room temperature. As used herein, unless otherwise defined, the term "room temperature" refers to a temperature of about 15°C to about 30°C, and preferably about 18°C to about 25°C. The amount of (R)-α-naphthylethylamine should be sufficient to obtain the enantiomerically enriched product. Preferably, the amount of (R)-α-naphthylethylamine is at least about 0.95 equivalents. Preferably, the (R)-α-naphthylethylamine is present in about 0.95 to 2 equivalents to the racemic modafinic acid and more preferably, the (R)-α-naphthylethylamine is present in an amount of about 1.1 equivalents.

Preferably, the combining step is performed under heating. The combining step can be heated at reflux temperature to facilitate the dissolution of the racemic modafinic acid. Preferably, the mixture is maintained at reflux temperature for a time sufficient to obtain a solution, such as at least one hour, more preferably, for about one hour to about 5-hours.

The converting step, step (b), may comprise adding an acid to the (R)-medafinic acid*(R)-α-naphthylethylamine salt to obtain a precipitate. Preferably, the acid is HCl. This step is preferably followed by isolation of the precipitate of (R)-modafinic acid*(R)-α-naphthylethylamine salt.

The process further comprises isolating the R-modafinic acid. The R-modafinic acid may be isolated by any method known in the art, for example, by filtering or decanting. The isolating step can include additional steps such as washing and drying the R-modafinic acid. Preferably, the isolated R-modafinic acid contains less than about 2% area by HPLC of the S-modafinic acid enantiomer. More preferably, the R-modafinic acid contains less than about 0.1% area by HPLC of the S-modafinic acid enantiomer.

Another embodiment of the invention encompasses R-modafinic acid containing less than about 2% area by HPLC of the S-modafinic acid enantiomer. More preferably, the R-modafinic acid contains less than about 0.1 % area by HPLC of the S-modafinic acid enantiomer.

Another embodiment encompasses a process for the optical resolution of racemic modafinic acid comprising: combining racemic modafinic acid, (-) α-methylbenzylamine and water at a temperature of less than about 50°C to obtain (R)-modafinic acid*α-methylbenzylamine salt as a precipitate; isolating the precipitate at a temperature of less than about 50°C; crystallizing the (R)-modafinic acid*α-methylbenzylamine salt from water; and adding an acid to obtain (R)-modafinic acid.

Preferably, the combining step is performed at a temperature of about 25°C to about 50°C, and more preferably, at a temperature of about 30°C to about 40°C. Optionally prior to the isolation step, the combination is stirred. The isolation may be performed by filtration and preferably, at a temperature of about 25°C to about 50°C. More preferably, the isolation step is performed at a temperature of at about 30°C to about 40°C. The crystallization is performed at a temperature of less than about 50°C, preferably, between about 25°C to about 50°C, and most preferably, the crystallization is performed at a temperature of about 30°C to about 40°C. The acid used in the process is HCl. Optionally, the (R)-modafinic acid may be further isolated, using methods such as filtration. The obtained R-modafinic acid contains less than about 2% area by HPLC of the S-modafinic acid enantiomer. More preferably, the R-modafinic acid contains less than about 0.1 % area by HPLC of the S-modafinic acid enantiomer.

Yet another embodiment of the invention encompasses a process for preparing an (R)-C₁₋₂ alkyl ester ofmodafinic acid comprising combining R-modafinic acid, at least one acidic reagent, and a solvent selected from the group consisting of: methanol/C₇-C₉-aromatic hydrocarbon, methanol/acetate ester, methanol and ethanol. The acidic reagent includes, but is not limited to, thionyl chloride, HCl, HBr or HI. Preferably, the acidic reagent is thionyl chloride or HCl. When the acidic reagent is HCl, typically the amount of HCl is from about 0.1 to 0.5 equivalents, and preferably about 0.25 equivalents. When the acidic reagent is thionyl chloride, typically it is added in an amount of about 0.1 to 3 equivalents and preferably, about 0.5 to 2.5 equivalents. When R-modafinic acid is combined with HCl or thionyl chloride and methanol, the product is the R-methyl ester of modafinic acid. Preferably, the C₇-C₉ aromatic hydrocarbon is toluene and the acetate ester is methylacetate or ethylacetate.

To facilitate the dissolution of the R-modafinic acid, the mixture of R-modafinic acid, acidic reagent, and solvent may be heated from about 40°C to about reflux. Preferably, when the acidic reagent is HCl, the mixture is heated at reflux. Typically, dissolution is accomplished after about 1 hour to about 3 hours of heating; however, large amounts of material may require more time. Stirring the mixture can aid dissolution. Once the R-modafinic acid is dissolved, the combination may be cooled to a temperature of about room temperature to about 0°C. Preferably, the combination is cooled to room temperature.

Preferably, the cooled mixture is maintained at room temperature for about 17 hours to about 24 hours. Optionally, the mixture may be stirred during cooling. The (R)-C₁₋₂ alkyl ester of modafinic acid may be isolated from the cooled mixture by any method known in the art, for example, precipitation, filtration, or solvent removal by evaporation.

Another embodiment of the invention encompasses a process for preparing armodafinil by preparing R-modafinic acid as described above and converting it to armodafinil. The process may be carried out consecutively (with isolation of the R-modafinic acid) or sequentially (without isolation). The process comprises combining racemic modafinic acid with (R)-α-naphthyl ethyl amine and acetone to obtain a solution, cooling the solution to obtain a precipitate of R-modafinic acid and reacting the R-modafinic acid with ammonia or ammonium hydroxide to obtain armodafinil. The conditions for preparing and, if desired, isolating R-modafinic acid are described above. The conditions for reacting the R-modafinic acid with ammonia or ammonium hydroxide to obtain armodafinil are the same as those described below for conversion of the C₁₋₂ ester R-modafinic to armodafinil.

Another embodiment of the invention encompasses a process for preparing (R)-C₁₋₂ alkyl ester of modafinic acid as described above and converting it to armodafinil. The process may be carried consecutively (with isolation of the ester) or concurrently (without isolation of the ester).

The concurrent process encompasses preparing armodafinil by a one-pot process from R-modafinic acid without separating the alkyl ester. This method is advantageous over known methods, in which the alkyl ester is separated and subsequently converted to armodafinil, because it contains fewer steps, and, thus, is streamlined and more suited for industrial application.

The one-pot process for preparing armodafinil comprises combining R-modafinic acid, at least one acidic reagent, and methanol or ethanol to obtain a solution of the C₁₋₂ ester R-modafinic and combining the solution of the ester with ammonia or ammonium hydroxide to obtain armodafinil. The conditions for the esterification are described above. As described above, the combination of R-modafinic acid with the acidic reagent in the presence of the solvent may be heated to reflux preferably for about three hours, to facilitate the dissolution of R-modafinic acid, followed by cooling the solution to about 0°C to room temperature. The ammonium hydroxide may be either an aqueous solution or produced by bubbling ammonia gas through the solution. Preferably, after the ammonia or ammonium hydroxide is added, the solution is stirred at room temperature for at least about 17 hours, although longer times may be necessary depending upon the amount of ester.

The resulting armodafinil may be isolated by any method known in the art, for example, precipitation followed by filtration, or solvent removal, such as evaporation, followed by trituration or recrystallization. The recovered armodafinil is preferably enantiomerically pure, containing less than about 0.5% area by HPLC of the S-enantiomer. More preferably, the armodafinil contains less than about 0.1% area by HPLC of the S-enantiomer.

Another embodiment of the invention encompasses armodafinil containing less than about 0.5% area by HPLC of the S-enantiomer. More preferably, the armodafinil contains less than about 0.1 % area by HPLC of the S-enantiomer.

Another embodiment of the invention encompasses armodafinil containing less than about 0.1 % area by HPLC of modafinic acid. Preferably, the armodafinil contains less than about 0.03% modafinic acid; and more preferably, less than about 0.02% modafinic acid.

Crude armodafinil may contain traces of starting modafinic acid due to hydrolysis of the methyl ester intermediate. Several methods for removing the traces of modafinic acid were tried and found to be ineffective including crystallizing armodafinil from ethanol, combining the armodafinil with inorganic bases, and exposing the armodafinil to basic activated carbon. Yet one process that succeeded was the use of activated basic or activated neutral alumina.

One embodiment of the invention encompasses a process for reducing the amount of modafinic acid in armodafinil comprising contacting a solution of armodafinil with activated basic or neutral alumina. The process comprises crystallizing armodafinil from ethanol in the presence of activated basic or activated neutral alumina (Al₂O₃). This process comprises forming a solution of armodafinil and ethanol, combining the solution with activated basic or neutral alumina to form a mixture, heating the mixture, and cooling to obtain a precipitate of armodafinil substantially free of modafinic acid. Typically, the term "substantially free of modafinic acid" means armodafinil containing less than about 0.1% area by HPLC of modafinic acid, preferably less than 0.03% modafinic acid, and more preferably less than about 0.02% modafinic acid. Optionally, the crystallization is done in the presence of an anti-solvent. Preferably, the anti-solvent is heptane or hexane.

Optionally, the armodafinil and ethanol are heated to promote dissolution of the armodafinil. Preferably, the armodafinil and ethanol are heated to the reflux temperature of the ethanol to promote dissolution of the armodafinil. Preferably, the ethanol is absolute ethanol. Typically, the alumina is present in an amount of about 1% to about 30% by weight of the armodafinil, and preferably, the alumina is present in an amount of about 5% to about 20% by weight of the armodafinil. Preferably, before cooling, the reaction mixture is maintained for about 30 minutes to about 7 hours.

The obtained armodafinil substantially free of modafinic acid may be further isolated by any method known to one skilled the art. Such methods include, but are not limited to, filtering and decanting. The isolating step can further comprise drying the precipitate, such as drying in a vacuum oven at a temperature of about 50°C at a pressure below about 100 mm Hg.

During the preparation of armodafinil an impurity bis(diphenylmethyl) disulfide ("R-MDF-DS") may be formed. R-MDF-DS has the following structure:

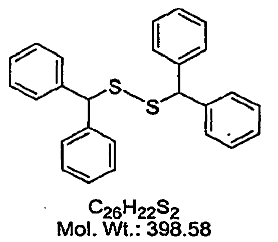

Another embodiment of the invention encompasses a process for preparing R-MDF-DS comprising combining diphenylmethanethiol acetate, a strong base and a aprotic polar solvent to obtain a suspension and adding water to the suspension to obtain R-MDF-DS.

Typically, the strong base is at least one of sodium methoxide, sodium ethoxide, sodium hydride, sodium hydroxide, sodium tert-butoxide, potassium methoxide, potassium ethoxide, potassium hydride, potassium hydroxide, or potassium tert-butoxide.

Preferably, the aprotic polar solvent is selected from the group consisting of: N,N-dimethylformamide and dimethylacetamide. Preferably, water is added to the suspension after about 3 days to about 5 days, and most preferably, the suspension is stirred during this time. In one even more preferable embodiment, the stirring is vigorous.

Preferably, after the water addition the suspension is stirred. Preferably, the stirring is performed for about 1 hour to about 3 hours, and more preferably, for about 1 hour. Optionally, after the water addition, the R-MDF-DS is isolated, *e.g.* by filtration. Preferably, the isolated R-MDF-DS is washed and dried. When washed, preferably, the isolated R-MDF-DS is washed with water. The drying may be performed at a temperature of about 50°C under reduced pressure.

Another embodiment of the invention encompasses armodafinil containing less than about 0.2% area by HPLC of R-MDF-DS. Preferably, the armodafinil contains less than about 0.1 % R-MDF-DS; and more preferably, less than about 0.05% area by HPLC of R-MDF-DS.

Another embodiment of the Invention encompasses a process for reducing the amount of R-MDF-DS in armodafinil comprising suspending armodafinil in C₅ to C₁₂ hydrocarbon. Preferably, the C₅ to C₁₂ hydrocarbon is heptane or hexane. Optionally, the suspension may be stirred while at about room temperature. The process may further comprise an isolation step. Preferably, the isolation is by filtration. The isolated product may be further washed and dried. Preferably, the washing step is performed with heptane. The drying may be carried out at about 50°C and under reduced pressure. Preferably, the obtained armodafinil contains less than about 0.2% R-MDF-DS; more preferably, less than about 0.1% R-MDF-DS; and most preferably, less than about 0.05% by area HPLC of R-MDF-DS.

Another embodiment of the invention encompasses pharmaceutical compositions comprising a therapeutically effective amount of armodafinil and a pharmaceutically acceptable excipient, wherein the armodafinil contains less than about 0.1 % area by HPLC ofmodafinic acid. Preferably, the armodafinil contains less than about 0.03% area by HPLC of modafinic acid, and more preferably less than 0.02% area by HPLC ofmodafinic acid. Another embodiment of the invention encompasses a process for preparing a pharmaceutical formulation comprising mixing a therapeutically effective amount of armodafinil and a pharmaceutically acceptable carrier, wherein the armodafinil contains less than about 0.1 % area by HPLC of modafinic acid. Preferably, the armodafinil contains less than about 0.03% area by HPLC of modafinic acid, and more preferably less than 0.02% area by HPLC of modafinic acid.

Another embodiment of the invention encompasses pharmaceutical compositions comprising a therapeutically effective amount of armodafinil and a pharmaceutically acceptable excipient, wherein the armodafinil contains less than about 0.5% area by HPLC of S-modafinil. Preferably, the armodafinil contains less than about 0.1% area by HPLC of S-modafinil. Another embodiment of the invention encompasses a process for preparing a pharmaceutical formulation comprising mixing a therapeutically effective amount of armodafinil and a pharmaceutically acceptable carrier, wherein the armodafinil contains less than about 0.5% area by HPLC of S-modafinil. Preferably, the armodafinil contains less than about 0.1% area by HPLC of modafinil.

Another embodiment of the invention encompasses pharmaceutical compositions comprising a therapeutically effective amount of armodafinil and a pharmaceutically acceptable excipient, wherein the armodafinil contains less than about 0.2% area by HPLC of R-MDF-DS. Preferably, the armodafinil contains less than about 0.1% area by HPLC of R-MDF-DS. Another embodiment of the invention encompasses a process for preparing a pharmaceutical formulation comprising mixing a therapeutically effective amount of armodafinil and a pharmaceutically acceptable carrier, wherein the armodafinil contains less than about 0.2% area by HPLC ofR-MDF-DS. Preferably, the armodafinil contains less than about 0.1 % area by HPLC of R-MDF-DS.

Pharmaceutical compositions may be prepared as medicaments to be administered orally, parenterally, rectally, transdermally, buccally, or nasally. Suitable forms for oral administration include, but are not limited to, tablets, powders, compressed or coated pills, dragees, sachets, hard or gelatin capsules, sub-lingual tablets, syrups, and suspensions. Suitable forms of parenteral administration include, but are not limited to, an aqueous or non-aqueous solution or emulsion. Suitable forms for rectal administration, include, but are not limited to, suppositories with hydrophilic or hydrophobic vehicle. For topical administration, suitable forms include, but are not limited to, suitable transdermal delivery systems known in the art, such as patches and for nasal delivery, suitable forms include aerosol delivery systems known in the art.

In addition to the active ingredient(s), the pharmaceutical compositions of the invention may contain one or more excipient or adjuvant. Selection of excipients and the amounts to use may be readily determined by the formulation scientist based upon experience and consideration of standard procedures and reference works in the field.

Excipients such as diluents increase the bulk of a solid pharmaceutical composition, and may make a pharmaceutical dosage form containing the composition easier for the patient and care giver to handle. Diluents for solid compositions include, but are not limited to, microcrystalline cellulose (e.g., AVICEL^{®}), microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g., EUDRAGIT^{®}), potassium chloride, powdered cellulose, sodium chloride, sorbitol, or talc.

Solid pharmaceutical compositions that are compacted into a dosage form, such as a tablet, may include, but are not limited to, excipients whose functions include, but are not limited to, helping to bind the active ingredient and other excipients together after compression, such as binders. Binders for solid pharmaceutical compositions include, but are not limited to, acacia, alginic acid, carbomer (e.g., CARBOPOL^{®}), carboxymethylcellulose sodium, dextrin, ethyl cellulose, gelatin, guar gum, hydrogenated vegetable oil, hydroxyethyl cellulose, hydroxypropyl cellulose (e.g., KLUCEL^{®}). hydroxypropyl methyl cellulose (e.g., METHOCEL^{®}), liquid glucose, magnesium aluminum silicate, maltodextrin, methylcellulose, polymethacrylates, povidone (e.g., KOLLIDON^{®}, PLASDONE^{®}), pregelatinized starch, sodium alginate, or starch.

The dissolution rate of a compacted solid pharmaceutical composition in the patient's stomach may be increased by the addition of a disintegrant to the composition. Excipients which function as disintegrants include, but are not limited to, alginic acid, carboxymethylcellulose calcium, carboxymethylcellulose sodium (e.g., AC-DI-SOL^{®}, PRIMELLOSE^{®}), colloidal silicon dioxide, croscarmellose sodium, crospovidone (e.g., KOLLIDON^{®}, POLYPLASDONE^{®}), guar gum, magnesium aluminum silicate, methyl cellulose, microcrystalline cellulose, polacrilin potassium, powdered cellulose, pregelatinized starch, sodium alginate, sodium starch glycolate (e.g., EXPLOTAB^{®}), or starch.

Glidants can be added to improve the flowability of a non-compacted solid composition and to improve the accuracy of dosing. Excipients that may function as glidants include, but are not limited to, colloidal silicon dioxide, magnesium trisilicate, powdered cellulose, starch, talc, or tribasic calcium phosphate.

When a dosage form such as a tablet is made by the compaction of a powdered composition, the composition is subjected to pressure from a punch and die. Some excipients and active ingredients have a tendency to adhere to the surfaces of the punch and die, which can cause the product to have pitting and other surface irregularities. A lubricant can be added to the composition to reduce adhesion and ease the release of the product from the die. Excipients which function as lubricants include, but are not limited to, magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc, or zinc stearate.

Flavoring agents and flavor enhancers make the dosage form more palatable to the patient. Common flavoring agents and flavor enhancers for pharmaceutical products that may be included in the composition of the invention include, but are not limited to, maltol, vanillin, ethyl vanillin, menthol, citric acid, fumaric acid, ethyl maltol, and tartaric acid.

Solid and liquid compositions may also be dyed using any pharmaceutically acceptable colorant to improve their appearance and/or facilitate patient identification of the product and unit dosage level.

In liquid pharmaceutical compositions of the invention, the active ingredient and any other solid excipients are suspended in a liquid carrier such as water, vegetable oil, alcohol, polyethylene glycol, propylene glycol, or glycerin.

Liquid pharmaceutical compositions may contain emulsifying agents to disperse uniformly throughout the composition an active ingredient or other excipient that is not soluble in the liquid carrier. Emulsifying agents that may be useful in liquid compositions of the invention include, but are not limited to, gelatin, egg yolk, casein, cholesterol, acacia, tragacanth, chondius, pectin, methyl cellulose, carbomer, cetostearyl alcohol, or cetyl alcohol.

Liquid pharmaceutical compositions of the invention may also contain a viscosity enhancing agent to improve the mouth-feel of the product and/or coat the lining of the gastrointestinal tract. Such agents include, but are not limited to, acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth, or xanthan gum.

Sweetening agents such as sorbitol, saccharin, sodium saccharin, sucrose, aspartame, fructose, mannitol, or invert sugar may be added to improve the taste.

Preservatives and chelating agents such as alcohol, sodium benzoate, butylated hydroxy toluene, butylated hydroxyanisole, or ethylenediamine tetraacetic acid may be added at levels safe for ingestion to improve storage stability.

According to the invention, a liquid composition may also contain a buffer such as gluconic acid, lactic acid, citric acid or acetic acid, sodium gluconate, sodium lactate, sodium citrate, or sodium acetate.

The solid compositions of the invention include, but are not limited to, powders, granulates, aggregates, and compacted compositions. The dosages include, but are not limited to, dosages suitable for oral, buccal, rectal, parenteral (including subcutaneous, intramuscular, and intravenous), inhalant, and ophthalmic administration. Although the most suitable administration in any given case will depend on the nature and severity of the condition being treated, the most preferred route of the invention is oral. The dosages may be conveniently presented in unit dosage form and prepared by any of the methods well known in the pharmaceutical arts.

Dosage forms include, but are not limited to, solid dosage forms like tablets, powders, capsules, suppositories, sachets, troches, and lozenges, as well as liquid syrups, suspensions, and elixirs. The dosage form of the invention may be a capsule containing the composition, preferably a powdered or granulated solid composition of the invention, within either a hard or soft shell. The shell may be made from gelatin, and, optionally, contain a plasticizer such as glycerin and sorbitol, and an opacifying agent or colorant. The active ingredient and excipients may be formulated into compositions and dosage forms according to methods known in the art.

A composition for tableting or capsule filling may be prepared by wet granulation. In wet granulation, some or all of the active ingredients and excipients in powder form are blended, and then further mixed in the presence of a liquid, typically water, that causes the powders to clump into granules. The granulate is screened and/or milled, dried, and then screened and/or milled to the desired particle size. The granulate may then be tabletted or other excipients may be added prior to tableting, such as a glidant and/or a lubricant.

A tableting composition may be prepared conventionally by dry blending. For example, the blended composition of the actives and excipients may be compacted into a slug or a sheet, and then comminuted into compacted granules. The compacted granules may subsequently be compressed into a tablet.

As an alternative to dry granulation, a blended composition may be compressed directly into a compacted dosage form using direct compression techniques. Direct compression produces a more uniform tablet without granules. Excipients that are particularly well suited for direct compression tableting include, but are not limited to, microcrystalline cellulose, spray dried lactose, dicalcium phosphate dihydrate, and colloidal silica. The proper use of these and other excipients in direct compression tableting is known to those in the art with experience and skill in particular formulation challenges of direct compression tableting.

A capsule filling of the invention may comprise any of the aforementioned blends and granulates that were described with reference to tableting, however, they are not subjected to a final tableting step.

Another embodiment of the invention encompasses a method of treating a patient suffering from excessive sleepiness associated with narcolepsy, shift work sleep disorder ("SWSD"), and/or obstructive sleep apnea/hypopnea syndrome ("OSA/HS") comprising administering to a patient in need thereof a therapeutically effective amount of armodafinil containing less than about 0.1% area by HPLC of modafinic acid. Preferably, the armodafinil contains less than about 0.03% area by HPLC of modafinic acid, and more preferably less than 0.02% area by HPLC of modafinic acid.

Having described the invention with reference to certain preferred embodiments, other embodiments will become apparent to one skilled in the art from consideration of the specification. The invention is further defined by reference to the following examples describing in detail the analysis of the armodafinil and intermediates thereof and methods for preparing thereof. It will be apparent to those skilled in the art that many modifications, both to materials and methods, may be practiced without departing from the scope of the invention.

### EXAMPLES

### HPLC methods:

### I. R-modafinic acid - Determination of Enantiomeric purity by HPLC:

The enantiomeric purity of R-modafinic acid was determined by high performance liquid chromatography (HPLC) under the following conditions. A Chiralpak AD-H column was used with 4.6*150mm DAIC 19324 packing. The eluent was a mixture of 90% heptane, 10% isopropyl alcohol, and 0.1% trifluoroacetic acid. The flow rate was 1.5 mL/min, the UV detector was set to 225 nm, and the column temperature was 25°C. The sample volume was 20 µL and the diluent was ethanol. The results were calculated as % area.

System suitability tests were performed before samples were analyzed. About 10 mg of modafinic acid (±) standard was accurately weighed in a 50 ml volumetric flask. The standard was dissolved and diluted with diluent using a sonicator to form a system suitability solution. The system suitability solution was injected into the column under the above-described conditions. Typical retention times were ∼7.5 minutes for S-modafinic acid and ∼9.5 minutes for R-modafinic acid. A resolution factor between the S-modafinic acid and R-modafinic acid peak not less than 1.8 was achieved.

Each sample was prepared by the following method. About 10 mg of modafinic acid sample was accurately weighed in a 50 mL volumetric flask. The sample was dissolved and diluted with diluent using a sonicator to form a sample solution.

### II. Armodafinil - Determination of Enantiomeric purity by HPLC

The enantiomeric purity of armodafinil was determined by high performance liquid chromatography (HPLC) under the following conditions. A Chiralpak AD-H column was used with 4.6* 150 mm DAIC 19324 packing. The eluent was a mixture of 80% heptane, 20% ethanol, and 0.1% trifluoroacetic acid. The flow rate was 1.5 mL/min, the UV detector was set to 225 nm, and the column temperature was 25°C. The sample volume was 20 µL and the diluent was ethanol. The results were calculated as % area.

System suitability tests were performed before samples were analyzed. About 10 mg of modafinil (±) standard was accurately weighed in a 20 ml volumetric flask. The standard was dissolved and diluted with diluent using a sonicator to form a system suitability solution. The system suitability solution was injected into the column under the above-described conditions. Typical retention times were ∼8.2 minutes for R-modafinil, and ∼11.1 minutes for S-modafinil. A resolution factor between the R-modafinil, and S-modafinil peak not less than 1.8 was achieved.

Each sample was prepared by the following method. About 10 mg of R-modafinil sample was accurately weighed in a 20 mL volumetric flask. The sample was dissolved and diluted with diluent using a sonicator to form a sample solution.

### III. Armodafinil - Determination of chemical purity by HPLC

The chemical purity of armodafinil was determined by high performance liquid chromatography under the following conditions. A YMC Basic 100 mm x 3.0 BA 99S031003QT column was used. The flow rate was 0.5 mL/min, the UV detector was set to 225 nm, and the column temperature was 25°C. The sample volume was 10 µL and the diluent was 60% water:45% acetonitrile. The results were calculated as % area.

Samples were eluted from the column by gradient elution with eluents A and B. The equilibration time was 5 minutes and the stop time was 20 minutes. Eluent A was 28% acetonitrile and 72% buffer, eluent B was 50% acetonitrile and 50% buffer, and the buffer was 0.02M KH₂PO₄ (adjusted to pH=4.0 with H₃PO₄). At time=0 minutes, 100% eluent A was used. At time=10 minutes, the eluent was a mixture of 70% eluent A and 30% eluent B. At time=20 minutes, the eluent was a mixture of 35% eluent A and 65% eluent B.

### Example 1: Use of (-) α-methylbenzylamine

Racemic modafinic acid (1 g) was charged in a 50 mL flask and (-) α-methylbenzylamine (0.27 g, 0.6 equivalents relative to modafinic acid) and 10.5 ml isopropanol were added to form a mixture. The mixture was stirred at reflux until dissolution to form a solution. The solution was cooled to room temperature and stirred overnight. Crystals precipitated from the solution. The crystals were filtered and washed with isopropanol. The crystals were found to contain (S)-modafinic acid*(S)-α-methyl benzyl amine salt in an amount of 18.7% area by HPLC.

### Example 2: Use of (-) α-methylbenzylamine with additional solvents

Example 1 was repeated using different solvents and temperatures. The results obtained are summarized in Table 1.

| Table 1. Preparation ofR-Modafinic Acid Using (-) α-methylbenzylamine | | | | |
|---|---|---|---|---|
| Experiment | Equivalents of amine | Temperature | Solvent | % (S)-modafinic acid*(S)-α-methyl benzyl amine salt by HPLC |
| 2a | 0.6 | reflux | isopropanol | 18.68 |
| 2b | 0.6 | reflux | ethyl acetate | 35.78 |
| 2c | 0.6 | reflux | acetone | 18.44 |
| 2d | 0.6 | room temperature | acetone | 15.4 |
| 2e | 0.55 | reflux | acetone/5% DMA | 4.5 |
| 2f | 0.55 | reflux | acetone/5% dimethyl formamide | 8.5 |

### Example 3: Use of L-proline

Racemic modafinic acid (7 g) was charged in a 2500 mL flask and L-proline (3.23 g) and 140 ml ethanol were added to form a mixture. The mixture was stirred at reflux until dissolution to form a solution. The solution was cooled to room temperature and stirred overnight. Crystals precipitated from the solution. The crystals were filtered and washed with ethanol. The crystals were found to contain (S)-modafinic acid*(S)-α-methyl benzyl amine salt in an amount of 19.7% area by HPLC.

### Example 4: Use of L-proline with additional solvents

Example 3 was repeated using 1.1 equivalents of L-proline relative to modafinic acid with solvents: water, acetone, isopropanol, and tetrahydrofuran. The results obtained are summarized in Table 2.

| Table 2. Preparation ofR-Modafinic Acid Using L-Proline | | | |
|---|---|---|---|
| Experiment | Solvent | Volume of Solvent (mL) | % (S)-modafinic acid*(S)-α-methyl benzyl amine salt by HPLC |
| 4a | water | 18 | 49.9 |
| 4b | acetone | 40 | 18.6 |
| 4c | ethanol | 20 | 17.3 |
| 4d | tetrahydrofuran | 20 | 15.5 |
| 4e | isopropanol | 20 | 29.9 |
| 4f | ethanol | 20 | 19.7 |

### Example 5: Use of α-Naphthyl Ethyl Amine

Racemic modafinic acid (1 g) was charged in a 100 mL flask and 1.06 g (1.7 eq.) of either (R)-α-naphthyl ethyl amine or (S)-α-naphthyl ethyl amine, and 10.5 ml acetone were added to form a mixture. The mixture was stirred at reflux until dissolution to form a solution. The solution was cooled to room temperature and stirred for two hours. Crystals precipitated from the solution. The crystals were collected by filtration and washed with acetone. The crystals were analyzed by HPLC and the results are contained in the following Table 3:

| Table 3. Preparation of R-Modafinic Acid Using α-Naphthyl Ethyl Amine | | | | |
|---|---|---|---|---|
| Experiment | Amine | Solvent | Volume of solvent (mL) | % (S)-modafinic acid*-α-naphthylethylamine salt by HPLC |
| 5a | (S)-α-naphthyl ethyl amine | acetone | 100 | 99.0 |
| 5b | (R)-α-naphthyl ethyl amine | acetone | 100 | 1.4 |

### Example 6: Preparation of R-methyl ester modafinic acid with HCl as reagent

A 50 ml flask was charged with R-modafinic acid (1.0 g), methanol (10 ml) and HCl 32% (0.1 ml, 0.25 eq.) to form a suspension. The suspension was stirred at reflux temperature for 1 hour, then cooled to room temperature to obtain a precipitate. The precipitated crystals were collected by filtration and washed with methanol.

### Example 7: Preparation of Modafinil with HCl as reagent

A 50 ml flask was charged with modafinic acid (1.0 g), methanol (10 ml) and HCl 32% (0.1 ml, 0.25 eq.) to form a suspension. The suspension was stirred at reflux temperature for 1 hour, then cooled to room temperature to obtain a clear solution. NH₄Cl (0.23 g, 1.2 eq.) and NH₄OH 25% (18 ml) were added to the solution and the solution was stirred at room temperature for 17 hours. Crystals precipitated from the solution. The crystals were collected by filtration and washed with water to obtain white crystals of racemic modafinil (0.5 g, 52.9%). (RMDF-DS: about 2.5% by HPLC).

### Example 8: Preparation of Modafinil with HCl as reagent

A 50 ml flask was charged with modafinic acid (1.0 g), methanol (5 ml) and HCl 32% (0.1 ml) to form a suspension. The suspension was stirred at room temperature for 19 hours to obtain a clear solution. NH₄Cl (0.23 g, 1.2 eq.) and NH₄OH 25% (18 ml) were added to the solution and the solution was stirred at room temperature for 24 hours. Crystals precipitated from the solution. The crystals were filtered and washed with water to obtain white crystals of racemic modafinil (0.66 g, 69.9%). (RMDF-DS: about 2.5% by HPLC).

### Example 9: Preparation of Armodafinil with 32% HCl

In the first step (step a), a 50 ml flask was charged with R-modafinil acid (5.0 g, 0.16% by HPLC S-modafinic acid), methanol (5 ml) and HCl 32% (0.1 ml) to form a suspension. The suspension was stirred at ambient temperature for 24 hours to obtain a crystalline precipitate, which were collected by filtration and analyzed to be the corresponding methyl ester.

In the second step (step b), the wet isolated methyl ester was mixed with methanol. Subsequently, ammonia (gas) was bubbled into the mixture for 30 min and the mixture was stirred for 12 hours. Precipitated crystals were collected and identified as armodafinil (yield by weight 66.7% ) containing 0.04% by HPLC of S-modafinil, purity: 98.6% by HPLC.

### Example 10: Preparation of Modafinil with thionyl chloride

A 100 ml flask was charged with modafinic acid (3.0 g) and methanol (50 ml) and cooled to 0°C. Thionyl chloride (1.63g, 1.7 eq.) was added drop wise. The reaction mixture was heated at 40°C for three hours and then cooled to room temperature. The solvent was evaporated from the reaction mixture and the methyl ester of modafinic acid was isolated. The methyl ester of modafinic acid was converted into modafinil by reaction with bubbling ammonia. (RMDF-DS: about 2.5% by HPLC)

### Example 11: Preparation of Armodafinil with Thionyl Chloride

A 100 ml flask was charged with R-modafinic acid (3.0 g) and methanol (50 ml) and cooled to 0°C. Thionyl chloride (0.8 g, 0.5 equivalents) was added drop wise. The reaction mixture was maintained at room temperature for at least 3 hours, and then cooled to 0°C. The methyl ester of modafinic acid was precipitated, filtered, and dried.

The isolated methyl ester ofmodafinic acid was mixed with methanol (5 ml/g) and ammonium hydroxide (15 ml/g) was added to the mixture. The mixture was stirred overnight and crystals precipitated from the mixture. The precipitated crystals were collected. The crystals obtained were determined to be armodafinil in 84.3% yield. The crystals were determined to contain 0.04% S-modafinil. (RMDF-DS: about 2.5% by HPLC).

### Example 12: Preparation of Armodafinil with thionyl chloride

In the first step, step a, a 100 ml flask was charged with R-modafinil acid (3.0 g), methanol (50 ml) and cooled to 0°C. Thionyl chloride (0.8 g, 0.5 equivalents) was added drop wise and the reaction mixture was maintained at room temperature during at least 3 hours, and then cooled to 0°C. The methyl ester of modafinic acid precipitated, collected by filtration, and dried.

In the second step, step b, the isolated ester was mixed with methanol and ammonium hydroxide was added. The mixture was stirred overnight and the precipitated crystals were collected. The obtained armodafinil (yield by weight 84.3%) contained 0.04% unwanted S-modafinil and had a purity of 98.4% by HPLC.

### Example 13: Purification of Armodafinil - removal of modafinic acid

Crude armodafinil (1.5 g) (obtained according to example 10) was mixed with 15 ml absolute ethanol and heated to reflux. When all the material was dissolved, basic alumina (10% w/w) was added to the solution. The suspension was heated during 10 min and filtered while hot. Then the solution was cooled to room temperature and the resulting crystals of armodafinil were filtered out. The wet material was dried at 50°C in a vacuum oven and analyzed. (RMDF-DS: about 0.1 % by HPLC)

The above-described procedure was repeated without the presence of alumina and in the presence of other additives as described in the following Tables 4 and 5:

| Table 4. Effect of Additives on the Removal of Modafinic Acid from Armodafinil | | | |
|---|---|---|---|
| Trial | Additive | % Modafinic Acid by HPLC | % Armodafinil ("MDF") by HPLC |
| | Starting crude armodafinil | 2.45 | 96.97 |
| 1 | No additive | 0.40 | 99.41 |
| 2 | Activated carbon SX1 20% | 0.55 | 99.34 |
| 3 | Tonsil 20% | 0.22 | 99.59 |
| 4 | Celite 20% | 0.39 | 99.45 |
| 5 | Basic Alumina 20% | 0.08 | 99.76 |

| Table 5. Effect of Additives on the Removal of Modafinic Acid from Armodafinil | | | |
|---|---|---|---|
| Trial | Additive | % Modafinic Acid by HPLC | % Armodafinil ("MDF") by HPLC |
| | Starting crude armodafinil | 0.32 | 99.05 |
| 1 | Neutral Al₂O₃ 20% | 0.02 | 99.79 |
| 2 | SiO₂ 20% | 0.26 | 99.59 |
| 3 | Activated carbon HB ultra 20% | 0.17 | 99.78 |
| 4 | Basic Al₂O₃ 20% | 0.03 | 99.76 |
| 5 | Celite 20% | 0.21 | 99.64 |
| 6 | No additive | 0.25 | 99.64 |

### Example 14: repetition of example la from '855: Resolution of 2-(benzhydrylsulfinyl)acetic acid (R-BHSO)

Modafinic acid (27.4 g) was suspended in 500 ml water and (-) α-methylbenzylamine (13 g, 1.08 equivalents) was added. The mixture was heated to reflux (100°C) and filtered hot. The solution was cooled to room temp (25°C) and the precipitated salt was filtered and the cake washed with water (300 ml). The S-modafinic acid percentage was 21.5% by HPLC. The product was recrystallized from water, the S-modafinic acid percentage was 1.5% by HPLC. A second recrystallization was carried out. The S-modafinic acid percentage was 0.4% by HPLC.

### Example 15: Preparation of Armodafinil

### (a) Resolution of racemic modafinic acid

To a 1L reactor modafinic acid (100 g) was added followed by 800 ml water. The mixture was heated to 40°C and then (-) α-methylbenzylamine (50.3 ml, 1.1 equivalents) was added drop wise. The mixture was stirred at 40°C for 1 hr, during which time a salt precipitated. The precipitated salt was filtered and the cake washed with water. The sample contained 6.5% by HPLC of S-modafinic acid ("S-BHSO"). The wet salt was recrystallized from water. The S-modafinic acid percent content was not more than 0.2%. The salt was hydrolyzed by adding 500 ml water to the wet salt followed by 20 ml HCl 32% at room temperature. The suspension was left to complete precipitation over 1-1.5 hr. R-modafinic acid is isolated by filtration. The material was dried at 50°C in a vacuum to obtain a constant weight. The S-modafinic acid content was 0.08% by HPLC.

### (b) Methylation -Amidation

A 2L reactor was charged with R-modafinic acid (70.0 g) and methanol (350 ml) and cooled to 5-10°C. Thionyl chloride (9.25 g, 0.5 equivalents) was added drop wise. The reaction mixture was maintained at 5-10°C for at least 20 hrs, then warmed to room temperature. The reaction was monitored by HPLC until less than 1% R-modafinic acid remained. Then 1050 ml ammonium hydroxide was added. The mixture was stirred overnight. The precipitated crystals were collected. Armodafinil crude was obtained (yield by weight 77.3%, R-modafinic acid: 0.25% by HPLC, RMDF-DS: about 2.5% by HPLC).

### (c) Purification

Crude armodafinil (53 g, from step (b) above) was mixed with 530 ml absolute ethanol and heated to reflux. Once the material dissolved, basic alumina (10% w/w) was added to the solution and the suspension was heated during 15 to 30 min and filtered while hot. Then the solution was cooled to room temperature and armodafinil crystallized. The crystallized armodafinil was filtered and the wet material was dried at 50°C in a vacuum oven and analyzed (yield 80-85 %, R-modafinic acid 0.08% by area HPLC, RMDF-DS: about 2.5% by HPLC).

### Example 16: Preparation of R-MDF-DS

A 250 ml round bottomed flask was charged with diphenylmethanthiol acetate (4.5 g), sodium methoxide (1.01 g, 1eq.) and N,N-dimethylformamide (45 ml). The suspension was stirred vigorously at 22 ± 2 °C for 3 days, while the flask was left open to air. A change in color was noticed from clear orange to brown to blue to green and a yellow suspension at the end. The suspension was analyzed in HPLC to get 70% ofR-MDF-DS. Water (100 ml) was added to obtain a heavy precipitation. The suspension was further stirred for 1 hour, filtered under reduced pressure, rinsed with water (20 ml) and dried in oven at 50°C under reduced pressure overnight to obtain grey to white solid of R-MDF-DS (1.5 g, 40.5%).

### Example 17: Purification of Armodafinil from R-MDF-DS with heptane

A 250 ml round-bottomed flask was charged with Armodafinil (20 g) and heptane (200 ml). The suspension was stirred at 22 ± 2 °C for 30 minutes then filtered under reduced pressure, rinsed with heptane (20 ml) and dried in an oven at 50 °C under reduced pressure overnight to obtain white crystals of armodafinil (RMDF-DS: less than 0.1% by HPLC).
Further aspects and features of the present invention are set out in the following numbered clauses.
1. A process for the optical resolution of racemic modafinic acid comprising crystallization of racemic modafinic acid with (R)-α-naphthylethylamine.
2. A process according to clause 1 comprising:
   (a) crystallization of racemic modafinic acid with (R)-α-naphthylethylamine to obtain (R)-modafinic acid*(R)-α-naphthylethylamine salt; and
   (b) converting the (R)-modafinic acid*(R)-α-naphthylethylamine salt to (R)-modafinic acid.
3. A process according to clause 2 wherein step (a) comprises combining racemic modafinic acid, (R)-α-naphthylethylamine and acetone to form a solution, and cooling the solution to obtain (R)-modafinic acid*(R)-α-naphthylethylamine salt as a precipitate, and optionally isolating the (R)-modafinic acid*(R)-α-naphthylethylamine salt.
4. A process according to clause 1 wherein comprising: combining racemic modafinic acid with (R)-α-naphthylethylamine and acetone to obtain a solution, cooling the solution to obtain a precipitate of (R)-modafinic acid*(R)-α-naphthylethylamine salt, and adding an acid.
5. A-process according to clause 3 or clause 4 wherein the solution is stirred during the cooling step.
6. A process according to clause 3 or clause 4 wherein the racemic modafinic acid, (R)-α-naphthylethylamine and a solvent are combined at a temperature of about room temperature.
7. A process according to any of clauses 3 to 5 wherein the solution is heated to about the reflux temperature before cooling.
8. A process according to any of clauses 3 to 7 wherein the (R)-α-naphthylethylamine is present in about 0.95 to about 2 equivalents relative to the racemic modafinic acid.
9. A process according clause 8 wherein the (R)-α-naphthylethylamine is present in an amount of about 1.1 equivalents relative to the racemic modafinic acid.
10. A process according to any of clauses 2 to 9, wherein step (b) comprises adding an acid to the (R)-modafinic acid*(R)-α-naphthylethylamine salt.
11. A process according clause 10 wherein the acid is HCl.
12. A process according clauses 1 to 11 further comprising isolating the R-modafinic acid.
13. A process according clauses 12 wherein the isolated R-modafinic acid contains less than about 2% area by HPLC of the S-modafinic acid enantiomer.
14. R-modafinic acid containing less than about 2% area by HPLC of S-modafinic acid.
15. The R-modafinic acid according to clause 14, wherein the R-modafinic acid contains less than about 0.1% area by HPLC of the S-modafinic acid.
16. A process for preparing an (R)-C₁₋₂ alkyl ester of modafinic acid comprising combining R-modafinic acid, at least one acidic reagent, and a solvent selected from the group consisting of: methanol/C₇-C₉ aromatic hydrocarbon, methanol/ acetate ester, methanol and ethanol.
17. A process according to clause 16 wherein the acidic reagent is selected from the group consisting of: thionyl chloride, HCl, HBr and HI.
18. A process according to clause 16 or clause 17, wherein the acidic reagent is thionyl chloride or HCl.
19. A process according to any of clauses 16 to 18, wherein the acidic reagent is HCl and is present in an amount from about 0.1 to 0.5 equivalents.
20. The process according to any of clauses 16 to 18, wherein the acidic reagent is thionyl chloride and is present in an amount from about 0.1 to 3 equivalents.
21. A process according to clause 16, wherein the combination of R-modafinic acid, methanol or ethanol, and the acidic reagent is heated to a temperature of about 40°C to about reflux.
22. The process according to clause 21, further comprising cooling the heated mixture at a temperature of about room temperature to 0°C.
23. The process according to any of clauses 16 to 22, further comprising isolating the (R)-C₁₋₂ alkyl ester of modafinic acid.
24. A process for preparing armodafinil comprising preparing R-modafinic acid in accordance with any of clauses 1 to 13 and converting it to armodafinil.
25. The process according to clause 24, wherein the R-modafinic acid is isolated prior to reacting with ammonia or ammonium hydroxide.
26. An one-pot process for preparing armodafinil comprising esterification of R-modafinic acid according to any of clauses 16 to 23 to form an (R)-C₁₋₂ alkyl ester of modafinic acid and reacting the (R)-C₁₋₂ alkyl ester of modafinic acid with ammonia or ammonium hydroxide to obtain armodafinil.
27. A process for preparing armodafinil comprising preparing (R)-C₁₋₂ alkyl ester in accordance with any of clauses 16 to 23 and converting it to armodafinil.
28. Armodafinil containing less than about 0.5% area by HPLC of S-modafinil enantiomer.
29. Armodafinil according to clause 28 containing less than about 0.1 % area by HPLC of S-modafinil enantiomer.
30. Armodafinil containing less than about 0.1% by HPLC area of modafinic acid.
31. Armodafinil according to clause 30 containing less than about 0.03% by HPLC area of modafinic acid.
32. A process for reducing the amount of modafinic acid in armodafinil comprising contacting a solution of armodafinil with activated basic or activated neutral alumina.
33. A process according to clause 32 comprising crystallizing armodafinil from ethanol in the presence of activated basic or activated neutral alumina.
34. A process according to clause 32 or 33 comprising forming a solution of armodafinil and ethanol, combining the solution with activated basic or neutral alumina to form a mixture, heating the mixture, and cooling to obtain a precipitate of armodafinil substantially free of modafinic acid.
35. The process according to any of clauses 32 to 34, wherein the alumina is present in an amount of about 1% to about 30% by weight of the armodafinil.
36. The process according to clause 35, wherein the alumina is present in an amount of about 5% to about 20% by weight of the armodafinil.
37. A process according to any of clauses 32 to 34, wherein the alumina is removed by filtration or decantation to obtain a solution.
38. A process according to any of clauses 34 to 37, wherein prior to the cooling step the reaction mixture is maintained for about 30 minutes to about 7 hours.
39. A process according to any of clauses 32 to 38 further comprising isolating the armodafinil having a reduced amount modafinic acid.
40. The process according to clause 39, wherein armodafinil product is further dried in a vacuum oven at a temperature of about 50°C and at a pressure below about 100 mm Hg.
41. A process according to any of clauses 32 to 40, wherein the armodafinil product is substantially free of modafinic acid.
42. Armodafinil containing less than about 0.2% area by HPLC of R-MDF-DS.
43. Armodafinil according to clause 42 containing less than about 0.1 % area by HPLC of R-MDF-DS.
44. Armodafinil according to clam 43 containing less than about 0.05% area by HPLC of R-MDF-DS.
45. A process for preparing R-MDF-DS comprising combining diphenylmethanthiol acetate, a strong base, and an aprotic polar solvent to obtain a suspension, maintaining, and adding water.
46. A process according to clause 45 wherein the maintaining step is while stirring.
47. A process according to clause 46 wherein the stirring is for about 3 days to 5 days.
48. A process according to clause 45 wherein after the water addition the suspension is stirred.
49. A process according to any of clauses 45 to 48, further comprising an isolation step wherein the isolation is by filtration.
50. A process for reducing the amount of R-MDF-DS in armodafinil comprising suspending it in C₅ to C₁₂ hydrocarbon.
51. A process according to clause 50, wherein the C₅ to C₁₂ hydrocarbon is heptane or hexane.
52. A process according to clause 50, wherein the suspension is stirred.
53. A process according to any of clauses 50 to 52 further comprising an isolation step.
54. A pharmaceutical composition comprising a therapeutically effective amount of armodafinil and a pharmaceutically acceptable excipient, wherein the armodafinil contains less than about 0.1 % area by HPLC of modafinic acid.
55. The pharmaceutical composition according to clause 54, wherein the armodafinil contains less than about 0.03% area by HPLC of modafinic acid.
56. A process for preparing a pharmaceutical formulation comprising mixing a therapeutically effective amount of armodafinil and a pharmaceutically acceptable carrier, wherein the armodafinil contains less than about 0.1 % area by HPLC of modafinic acid.
57. The process according to clause 56, wherein the armodafinil contains less than about 0.03% area by HPLC of modafinic acid.
58. The process according to clause 57, wherein the composition is a tablet, powder, compressed or coated pill, dragee, sachet, hard or gelatin capsule, sub-lingual tablet, syrup, or suspension.
59. A pharmaceutical composition comprising a therapeutically effective amount of armodafinil and a pharmaceutically acceptable excipient, wherein the armodafinil contains less than about 0.5% area by HPLC of S-modafinil.
60. The pharmaceutical composition according to clause 59, wherein the armodafinil contains less than about 0.1 % area by HPLC of S-modafinil.
61. A process for preparing a pharmaceutical formulation comprising mixing a therapeutically effective amount of armodafinil and a pharmaceutically acceptable carrier, wherein the armodafinil contains less than about 0.5% area by HPLC of S-modafinil.
62. The process according to clause 61, wherein the armodafinil contains less than about 0.1% area by HPLC of S-modafinil.
63. The process according to clause 61, wherein the composition is a tablet, powder, compressed or coated pill, dragee, sachet, hard or gelatin capsule, sub-lingual tablet, syrup, or suspension.
64. A pharmaceutical composition comprising a therapeutically effective amount of armodafinil and a pharmaceutically acceptable excipient, wherein the armodafinil contains less than about 0.2% area by HPLC of R-MDF-DS.
65. The pharmaceutical composition according to clause 64, wherein the armodafinil contains less than about 0.1% area by HPLC of R-MDF-DS.
66. A process for preparing a pharmaceutical formulation comprising mixing a therapeutically effective amount of armodafinil and a pharmaceutically acceptable carrier, wherein the armodafinil contains less than about 0.2% area by HPLC of R-MDF-DS.
67. The process according to clause 66, wherein the armodafinil contains less than about 0.1% area by HPLC of modafinic acid.
68. The process according to clause 67, wherein the composition is a tablet, powder, compressed or coated pill, dragee, sachet, hard or gelatin capsule, sub-lingual tablet, syrup, or suspension.
69. Use of a process according to any of clauses 24-27, 32-41, or 50-53 in the preparation of armodafinil.
70. Use of a process according to any of clauses 24-27, 32-41, or 50-53 in the preparation of armodafinil.

## Claims

1. A process for reducing the amount of bis(diphenylmethyl)disulfide in armodafinil comprising suspending it in a C₅ to C₁₂ hydrocarbon.

2. A process according to claim 1, wherein the C₅ to C₁₂ hydrocarbon is heptane or hexane.

3. A process according to claim 1 or claim 2, wherein the suspension is stirred.

4. A process according to any preceding claim, wherein the process is performed at room temperature.

5. A process according to any preceding claim, further comprising an isolation step.

6. A process according to claim 5, wherein the isolation is by filtration.

7. A process according to claim 5 or claim 6, wherein after isolation, the product is further washed and dried.

8. A process according to claim 7, wherein the washing step is performed with heptane.

9. A process according to claim 7 or claim 8, wherein the drying step is carried out at about 50 °C under reduced pressure.

10. Use of a process according to any of claims 1 to 9 in the preparation of armodafinil.
